Europäisches Patentamt

**European Patent Office**

Office européen des brevets

(19)

(11) **EP 0 714 629 A1**

(12) **EUROPEAN PATENT APPLICATION**
published in accordance with Art. 158(3) EPC

(43) Date of publication:
05.06.1996 Bulletin 1996/23

(21) Application number: 95919624.7

(22) Date of filing: 24.05.1995

(51) Int. Cl.$^6$: **A61B 5/04**

(86) International application number:
PCT/JP95/00993

(87) International publication number:
WO 95/31931 (30.11.1995 Gazette 1995/51)

(84) Designated Contracting States:
**DE FR GB NL SE**

(30) Priority: 25.05.1994 JP 110629/94
22.05.1995 JP 122064/95

(71) Applicant: **MOTOYAMA, Hiroshi**
**Mitaka-shi Tokyo 181 (JP)**

(72) Inventors:
• **MOTOYAMA, Hiroshi**
**Mitaka-shi, Tokyo 181 (JP)**

• **KOBAYASHI, Keisuke**
**Miyamae-ku, Kawasaki-shi, Kanagawa 21 (JP)**
• **KINOSHITA, Toshimasa**
**Mitaka-shi, Tokyo 181 (JP)**

(74) Representative: **Downey, William Gerrard et al**
**Wilson, Gunn, M'Caw,**
**41-51 Royal Exchange,**
**Cross Street**
**Manchester M2 7BD (GB)**

(54) **BIOLOGICAL SURFACE POTENTIAL MEASURING DEVICE AND DIAGNOSING DEVICE**

(57) A device for measuring the surface potential of a living body, and a device for diagnosing the living body on the basis of the measured surface potential. The diagnosing device comprises a biological surface potential measuring device which is adapted to be pressed against a surface of the living body and has a pickup composed of a vibrating reed potential sensor, a processing device for taking in measurements from the biological surface potential measuring device to calculate optimum values, make radar charts and bar graphs from the optimum values and to diagnose abnormality of the living body, and a display and a printer for displaying and outputting information which are determined by the processing device.

FIG. 3

## Description

Technical Field

The present invention generally relates to a biological surface potential measuring device for measuring a specific potential gradient on the surface of a living body, and particularly to a diagnosing device comprising the measuring device for analyzing an electric field developed on the living body and detecting normality or abnormality of a biological energy field for preventive medical treatment.

Background Art

It has been well-known in Oriental medicine that a variation of the surface of a living body is observed to diagnose the living body. The inventors of this patent application found that an electric field on the surface of a living body is closely related to normality or abnormality of a biological energy field. The inventors furthermore have recognized that preventive medical treatment may be subjected to a patient by measuring the surface potential of his body.

Conventionally, two electrodes have been attached to the surface of a living body and the potential difference therebetween has been measured by means of a differential amplifier to get a biological surface potential (see Japanese patent laid-open No. 3-268738).

In such a conventional biological surface potential measuring device, two electrodes must be attached on the measuring points of a living body every when measuring is carried out. The attachment of electrodes is troublesome, and in some cases is difficult depending upon the location of measuring point. Also, it takes a lot of time to attach electrodes when the number of measuring points is large. The conventional measuring device furthermore has such a defect that precise measurements are not obtained, because two electrodes are directly attached on the surface of a living body and voltage is applied therebetween, thereby resulting in polarization of the surface of a living body. This polarization makes measurements different from real values.

Also, the biological surface potential measuring by the conventional device has following problem. That is, operators or inspectors for the device are limited to those who have thorough knowledge for the items to be measured, because a living body is constantly active so that measurements are always varied, as a result of which optimum values among measurements must be selected for diagnosing.

Disclosure of Invention

The object of the present invention is to provide a biological surface potential measuring device by means of which any points on the surface of a living body may be measured easily and precisely.

Another object of the present invention is to provide a device using the measuring device for diagnosing physical constitution and condition to obtain useful information for health care by calculating optimum values from measurements supplied by the measuring device.

The further object of the present invention is to provide a diagnosing device in which useful information for health care may be displayed visually.

According to the present invention, a device for measuring the surface potential of a living body comprises a pickup composed of a vibrating reed potential sensor for producing an AC voltage signal, the pickup being contacted to a surface of the living body, and a detecting circuit for detecting the surface potential of the living body from the AC voltage signal supplied by the pickup.

The pickup includes a detecting electrode, a vibrator for vibrating the detecting electrode, a resistor connected to the detecting electrode, across thereof the AC voltage signal is generated. The detecting circuit includes a bandpass filter for removing frequency components other than vibrating frequency generated by the vibrator from the AC voltage signal, an AC amplifier for amplifying the AC voltage signal from the bandpass filter, an oscillator for generating an oscillating voltage to vibrate the vibrator of the pickup, a reference clock generator for generating a reference clock on the basis of the oscillating voltage supplied by the oscillator, a rectifier, to which the amplified AC voltage signal and the reference clock are supplied, for synchronous-detecting the amplified AC voltage signal on the basis of the reference clock to generate a synchronous-detected signal and integrating the synchronous-detected signal to detect the surface potential on the surface of the living body, and a DC amplifier for amplifying the detected potential to output it as an analog measurement.

A diagnosing device according to the present invention comprises the above-described measuring device for measuring the surface potential of a living body and generating the measured surface potential as analog measurements, a processing device for obtaining predetermined information from the surface potentials measured on a plurality of measuring points by the device for measuring the surface potential of the living body, a display for indicating the information on a screen, and a printer for printing the information.

The processing device includes, an A/D converter for converting the analog measurements into digital measurements, a sampling unit for sampling the predetermined number of the digital measurements every predetermined time interval, an optimum value calculating unit for calculating optimum values on the basis of the sampled digital measurements, a memory for storing the optimum values, a graph forming unit for reading out the optimum values stored in the memory to form graphs, and a diagnosing unit for diagnosing the living body on the basis of the optimum values read out from the memory.

Brief Description of Invention

Fig. 1 is a circuit diagram of an embodiment of a biological surface potential measuring device according to the present invention.
Fig. 2 is a circuit diagram of a part of the rectifier shown in Fig. 1.
Fig. 3 shows the structure of the pickup.
Fig. 4 is a block diagram of a diagnosing device according to the present invention.
Fig. 5 is a functional block diagram of the processing device.
Fig. 6 shows "Seiketsu" at the tips of fingers and toes.
Fig. 7 is a flow chart of the process in the processing unit.
Fig. 8 is a flow chart of the preprocess.
Fig. 9 is a flow chart of input of subject's name.
Fig. 10 is a flow chart of measuring for biological surface potential.
Fig. 11 is a flow chart of indicating process on the display.
Fig. 12 shows the order of circulation of "Keiraku's."
Fig. 13 shows an example of radar chart.
Fig. 14 shows an example of bar graph.
Fig. 15 is a flow chart of printing process.
Fig. 16 shows a list of measurements and abnormal "Keiraku's."

Best Mode for Carrying out the Invention

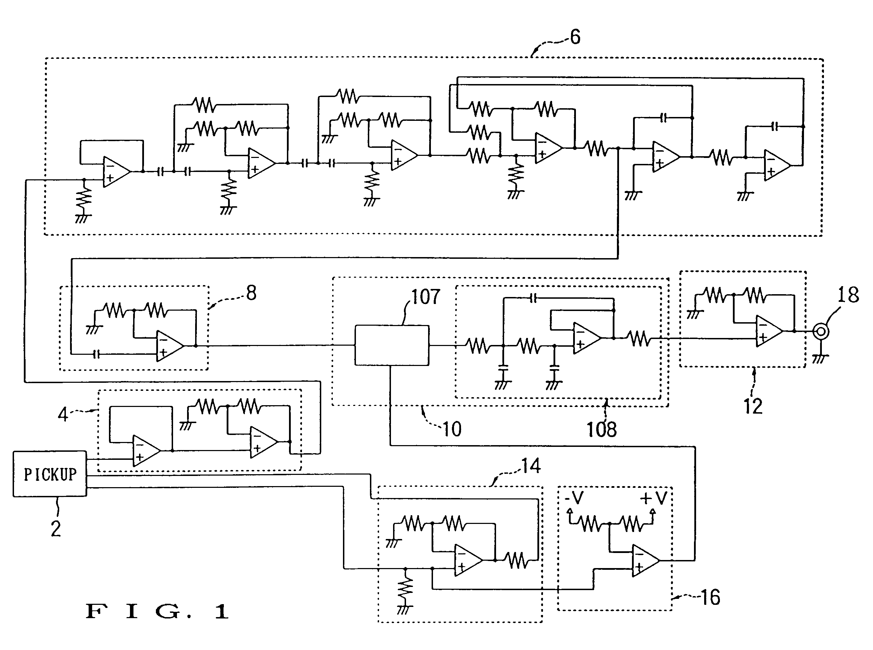

Fig. 1 shows a circuit diagram of an embodiment of the biological surface potential measuring device according to the present invention. The measuring device comprises a pickup 2 composed of a vibrating reed potential sensor, an impedance converter 4, a bandpass filter 6, an AC amplifier 8, a rectifier 10, a DC amplifier 12, an oscillator 14 and a reference clock generator 16.

The pickup 2 includes a vibrating detecting electrode, i.e., a vibrating reed to which a resistor is connected. An AC current flows through the resistor on the basis of varying capacitance between the detecting electrode and the surface of a living body so that an AC signal is generated across the resistor. The structure and operation of the pickup 2 will be explained in detail hereinafter.

The impedance converter 4 includes operational amplifiers and resistors so as to realize impedance matching between the pickup 2 and the bandpass filter 6.

The bandpass filter 6 includes operational amplifiers, resistors and capacitors so as to remove frequency components other than vibrating frequency generated by the detecting electrode.

The AC amplifier 8 amplifies an AC voltage passing through the bandpass filter 6 and supplies it to the rectifier 10.

The oscillator 14 includes an operational amplifier and resistors so as to generate an oscillating voltage to the pickup 2.

The reference clock generator 16 comprises a reference voltage generator including a comparator and resistors, an input (+) of the comparator being connected to an input (+) of the operational amplifier in the oscillator 14. The comparator in the reference clock generator 16 compares the oscillating voltage from the oscillator with a reference voltage to generate a reference clock synchronized with the oscillating voltage and supplies it to the rectifier 10.

The rectifier 10 includes a phase detector using a synchronous detecting system synchronized with the reference clock from the clock generator 16, and an integrating circuit 108 for converting an output including AC components from the phase detector 107 into a DC voltage.

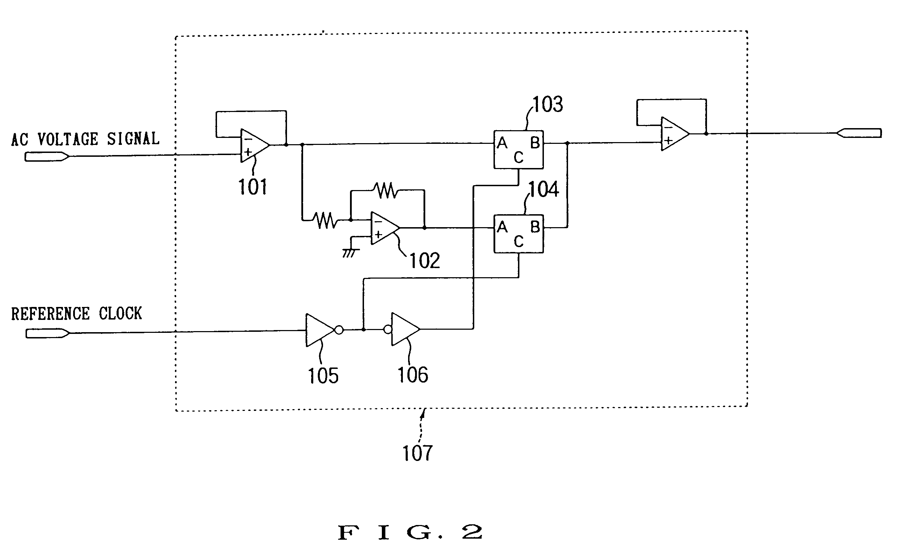

Fig. 2 shows a circuit diagram of the phase detector 107. The phase detector includes operational amplifiers 101, 102 to which the AC voltage signal from the AC amplifier 8 is supplied, two analog switches 103, 104 which are connected to the operational amplifiers respectively, inverters 105, 106 to which the reference clock from the clock generator 16 is supplied. The output from the phase detector 107 is supplied to the integrating circuit 108 in which the output is converted into the DC voltage.

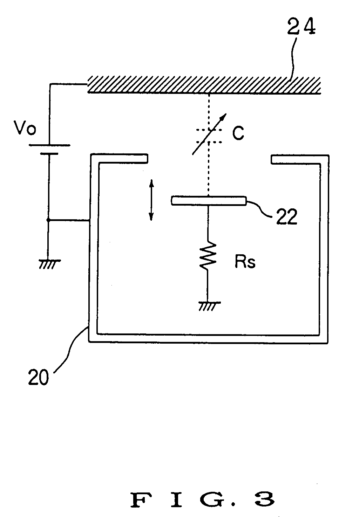

The structure and operation of the pickup 2 is now explained with reference to Fig. 3 which shows the structure of the pickup 2. The detecting electrode (vibrating reed) 22 which has the size of 1mm by 5mm is provided in a sensor case 20. This detecting electrode is vibrated in a very small amplitude at several hundreds Hz by a mechanical vibrator such as a piezoelectric vibrator (not shown).

When the detecting electrode 22 is approached to the surface of a living body 24, a capacitor is formed by the detecting electrode 22 and the living body 24. Charge Q which is represented by the following formula (1) is induced in the detecting electrode 22 depending on the potential difference $V_O$ with respect to the living body 24,

$$Q = C \cdot V_O \tag{1}$$

where C is capacitance of said capacitor.

When the detecting electrode 22 is caused to be vibrated at an angular frequency $\omega$, the capacitance C is varied as shown in the following formula (2),

$$C = C_O(1 + \alpha \sin \omega t) \tag{2}$$

where $\alpha$ is a coefficient decided by an amplitude of vibration.

When the detecting electrode is electrically connected to the ground through the resistor whose resistance is represented by $R_s$, the AC current which is represented by the following formula (3) is passed through the resistor.

$$I = \frac{dQ}{dt} = \frac{d}{dt}C_O(1 + \alpha \sin \omega t) \ V_O = \alpha \omega C_O V_O \cos \omega t \tag{3}$$

Therefore, the AC voltage signal $V_s$ represented by the following formula (4) is appeared across the resistor.

$$V_s = R_s \cdot \alpha \omega C_O V_O \cos \omega t \tag{4}$$

The potential $V_O$ on the surface of the living body 24 is obtained by synchronously detecting the AC voltage signal $V_s$.

The potential measuring method by this vibrating reed potential sensor is a measuring method in which an input impedance is infinite theoretically.

Returning to Fig. 1, the operation of the measuring device is now explained. The oscillator 14 supplies the oscillating frequency (or oscillating voltage) to the pick up 2 through positive feedback. The oscillating frequency drives the vibrator in the pickup 2. Next, the pickup 2 is contacted to a measuring point on the skin of the living body, the distance between the detecting electrode 22 and the skin being about 2mm. As described above, the AC voltage signal $V_s$ is output from the pickup 2 and is supplied to the bandpass filter 6 through the impedance converter 4.

In the bandpass filter 6, extra frequency components are removed, and then only the AC voltage signal $V_s$ is passed and supplied to the AC amplifier 8. The AC voltage signal $V_s$ is amplified in the AC amplifier and is supplied to the rectifier 10.

The reference clock is also supplied to the rectifier 10, which is generated by comparing the oscillating voltage from the oscillator 14 with the reference voltage in the comparator of the reference clock generator 16.

As described above, the rectifier 10 includes the phase detector 107 using a synchronous detecting system and the integrating circuit 108. In the rectifier 10, the AC voltage signal is rectified to obtain the potential $V_O$ on the skin of the living body. The potential $V_O$ is amplified in the DC amplifier 12 and supplied from an output 18 as analog measurements.

According to the biological surface potential measuring device as described before, measuring can be easily effected only by contacting the pickup to measuring points on the skin, while electrodes are to be attached on the skin in the conventional measuring device. Furthermore, precise measurements are obtained according to the present invention, because a voltage is not applied to the skin so that polarization is not occurred in the skin.

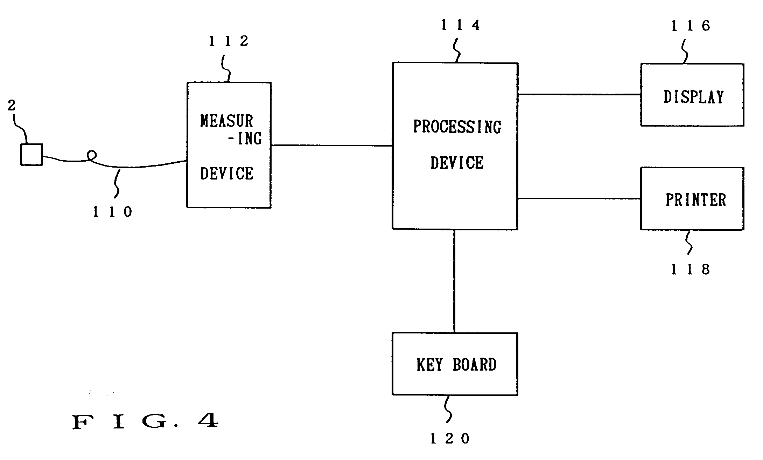

A diagnosing device using above-described biological surface potential measuring device is now explained. Fig. 4 shows a block diagram of the diagnosing device according to the present invention. This diagnosing device comprises the biological surface potential measuring device 112 connected through a code 110, a processing device 114 for fetching the measurements from the measuring device 112, a display 116, a printer 118 and a key board 120. The display, the printer and the key board are connected to the processing device respectively.

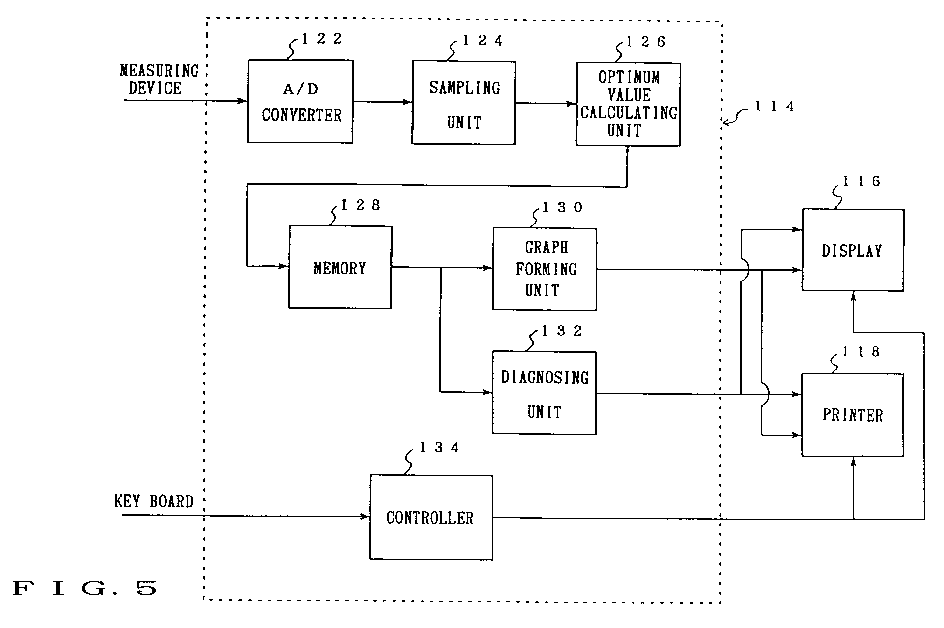

Fig. 5 shows a functional block diagram of the processing device 114. The processing device 114 includes an A/D converter 122 for converting analog measurements from the measuring device into digital measurements, a sampling unit 124 for sampling the predetermined number of digital measurements every constant time interval, an optimum value calculating unit 126 for calculating optimum values from the sampled measurements, a memory 128 for storing the calculated optimum values, a graph forming unit 130 for generating data to form radar charts and bar graphs on the basis of the calculated optimum values from the memory, a diagnosing unit 132 for diagnosing "Keiraku (the meaning thereof is described later in detail)" in Oriental medicine on the basis of the calculated optimum values from the memory, and a controller 134 for controlling the above-described elements, the display 116 and the printer 118. In order to simplify the drawing, only controlling lines from the controller to the display 116 and the printer 118 are shown in Fig. 5.

Next, the operation of the diagnosing device is explained. In this diagnosing device, "Seiketsu" points which are defined in Oriental medicine are used as measuring points for biological surface potential. The "Seiketsu" points are at the tips of fingers and toes, and "Keiraku" starts from "Seiketsu" point. It is well-known that these points represent well the state of "Keiraku." Physical constitution, physical condition and the like based on Oriental medicine may be diagnosed by measuring the surface potential on "Seiketsu" points.

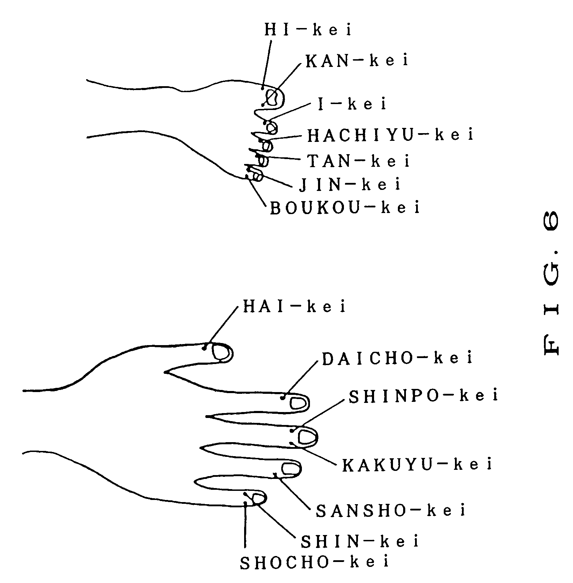

Measuring for surface potential is effected for 24 points in total consisting of "Seiketsu" points for usual "Keiraku" called as "Sei-12-kei" (24 routes in total in right and left halves of a body) and "Seiketsu" points for special "Keiraku" called as "Kakuyu-kei" and "Hachiyu-kei" (4 routes in total in right and left halves of a body). Fig. 6 shows "Seiketsu" at the tips of fingers and toes. Each "Seiketsu" points has been named of related "Keiraku."

The pickup is contacted in turn to 28 "Seiketsu" points to measure the biological surface potential on each "Seiketsu" point by means of the biological surface potential measuring device 112. The processing device 114 fetches the analog measurements of biological surface potential from the measuring device 112. In the processing device 114, data for the displya and printer is generated. The data is supplied to the display 116 and/or the printer 118 where radar charts, bar graphs and the like are displayed and/or printed.

The function of the processing device 114 is now explained with reference to the functional block diagram of Fig. 5 and flaw charts. As described before, a living body is always active so that biological surface potential is constantly varied. The processing device has a function of calculating optimum values from the varying measurement, as a result of which anyone can easily get necessary information useful for health care. The calculation of the optimum values is effected by using the method of weighted mean in which the newest measurement within a plurality of measurements is most heavily weighted and the older measurement is lightly weighted in order to soften the variation of the measurements.

The processing device 114 further has a function of displaying diagnosis result in accordance with the diagnosis of physical constitution and condition to get easily the information useful for health care.

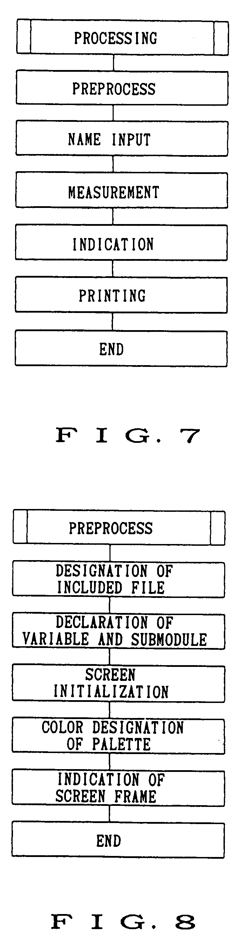

The processing device 114 has two important functions as described above. As shown in a flow chart of Fig. 7, the process of performing the two functions consisting of five process, i.e. a preprocess, a subject's name input process, a measuring process, a displaying process and a printing process. These processes are effected under the control of the controller 134.

As shown in the flow chart of Fig. 8, the preprocess comprises the steps; the designation of included file, the declaration of variable and submodule, the initialization of screen, the color designation of palette, and the indication of screen frame. These steps are generally necessary for preprocess. In the color designation of palette, a kind of color is designated. Finally, the frame of total screen is indicated.

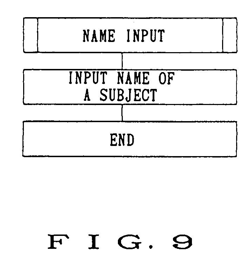

In the subject's name input process, the name of a subject is input from the key board 120 as shown in the flow chart of Fig. 9. The subject's name is printed together with measuring and diagnosing results.

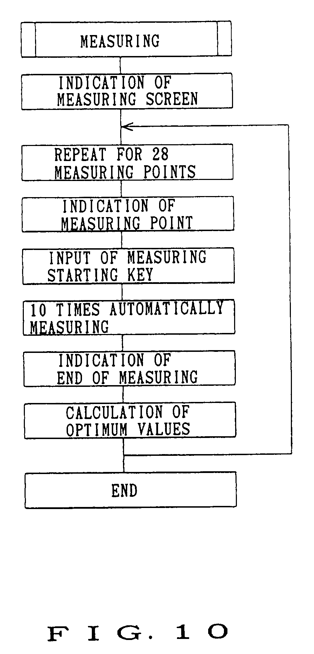

The measuring of biological surface potential is effected according to the flow chart shown in Fig. 10. When the operator or inspector of the biological surface potential measuring device presses the pickup 2 against the measuring point ("Seiketsu" point) which is designated on the display 116, the analog measurements are output from the measuring device and supplied to the processing device 114. When the start of measuring is designated from the key board 120, the A/D converter 122 converts the analog measurements into the digital measurements and supplies them to the sampling unit 124.

In the sampling unit 124, ten digital measurements are sampled for one "Seiketsu" point at predetermined time interval, for example 2 sec time interval. When the sampling process is terminated, the end of sampling is indicated on the display 10. The operator then can notice the end of measuring for one point.

Ten digital measurements are supplied to the optimum value calculating unit 126. As described before, the weighted mean is the method in which the newest measurement is most heavily weighted and the older measurement is lightly weighted to soften the variation of the measurements. As a result, the optimum value is calculated.

The weighted mean is now explained in more detail. According to the weighted mean, n measurements obtained by one time measuring are averaged in such a manner that a weigh "1" is added to the (n)th measurement, a weight "k" to the (n-1)th measurement, a weight "k$^2$" to the (n-2)th measurement and so on, where $k=\omega/(\omega+1)$, $\omega$ being a coefficient. Assuming that the (i)th measurement is $x_i$ and the optimum value is $S_n$, the optimum value is given by the following formula (5),

$$S_n = \sum_{j=1}^{n} X_j \times k^{n-j} / \sum_{j=1}^{n} k^{n-j} \tag{5}$$

where $k=1/2$ ($\omega=1$). The optimum value calculated in this manner is stored in the memory 130.

The process described above is repeated to all the measuring points, for example 28 points, so that the optimum values for all of the measuring points are stored in the memory 128.

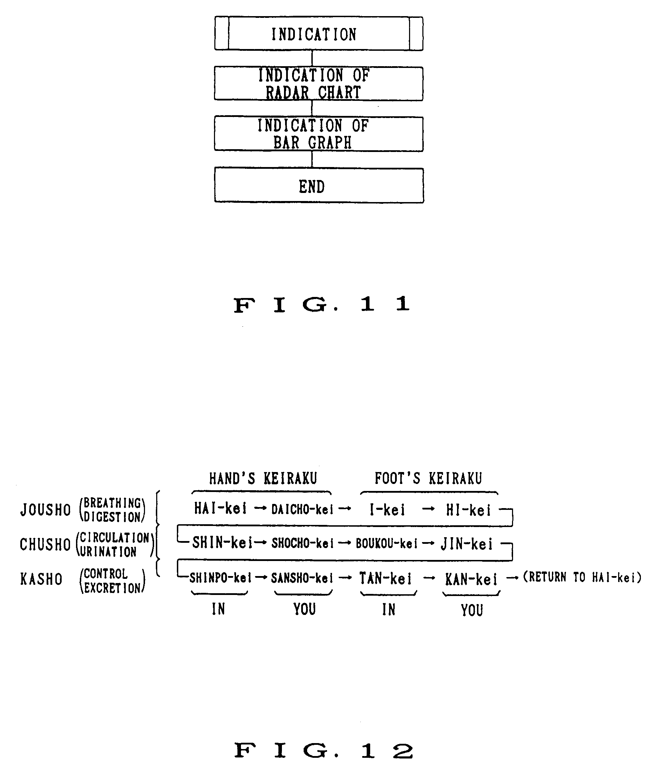

The graph forming unit 130 reads out the optimum values for each of 28 measuring points from the memory, and forms graphs such as radar chart and bar graph according to the flow chart in Fig. 11, which are indicated visually on the display 116. These radar chart and bar graph are indicated according to the theory of three positive-three negative "3-In-3-You" in Oriental medicine (i.e. the theory for diagnosing of physical constitution and condition).

The diagnosing unit 132 diagnoses "Keiraku" desired to be cured on the basis of the optimum values read out from the memory 128. According to Oriental medicine, it is thought that the 12 "Keiraku's" are conducted to the tips of hands and feet and circulated with their ends succeedingly connected. These "Keiraku's" are classified into Negative ("In") and Positive ("You").

The relationship of these "Keiraku's" is complex as shown in Fig. 12, so it is not easy to judge the measured results in accordance with the classification of "In" and "You", that of right and left of hands and feet, and that of functions of internal organs. Therefore, the inventors of this patent application intend to have radar charts and bar graphs had the following functions.

Radar chart: The balance of hands and feet, that of right and left, and that of "In" and "You" are visually indicated, thereby allowing the balance to be checked.

Bar graph: The 12 "Keiraku's" are classified for the functions of breathing, digestion, circulation, urination, control and excretion, then the magnitude of measurement per classified "Keiraku" is visually indicated, thereby allowing the functions to be checked. "Keiraku's" are arranged in the bar graph along the circulated loop of a series of "Keiraku's" and indicated so as to judge which "Keiraku" has abnormality.

Depend upon these indications, it becomes easy to diagnose "Keiraku" from various view points due to Oriental medicine.

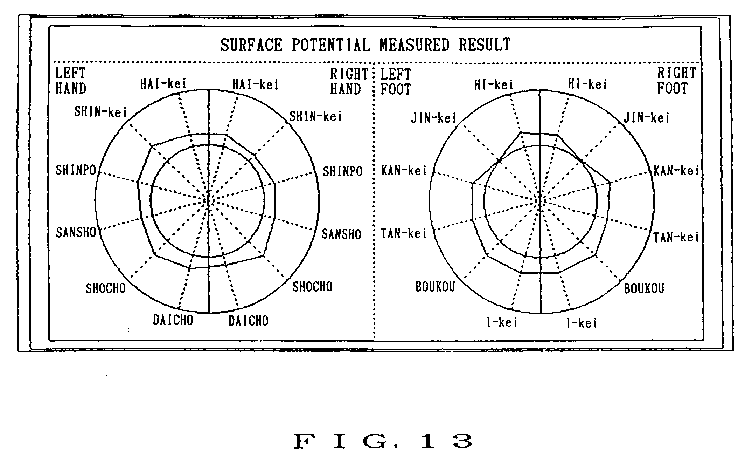

An example of radar chart is shown in Fig. 13 i which two radar charts are indicated on both sides. The radar chart on left side shows the optimum values for "Keiraku's" of the upper half of a body ("Hai-kei", "Shin-kei", "Shinpo-kei", "Sanshokei", "Shocho-kei", "Daicho-kei"), while the radar chart on right side shows the optimum values for "Keiraku's" of the lower half of a body ("Hi-kei", "Jin-kei", "Kan-kei", "Tan-kei", "Boukou-kei", "I-kei"). In each radar chart, "Keiraku's" belonged to the left side of a body are indicated on a left half, while "Keiraku's" belonged to the right side of a body are indicated on a right half. In the radar chart on left side, "Keiraku's" belonged to "In" ("Hai-kei, "Shin-kei" and "Shinpo-kei") are indicated on a upper half, while "Keiraku's" belonged to "You" ("Sansho-kei", "Shocho-kei" and "Daicho-kei") are indicated on a lower half. In the radar chart on right side, "Keiraku's" belonged to "In" (Hi-kei, Jin-kei and Kan-kei) are indicated on a upper half, while "Keiraku's" belonged to "You" (Tan-kei, Boukou-kei and I-kei) are indicated on a lower half. In each of radar charts, two "Keiraku's" having a relationship of "In" - "You", for example "Hai-kei" and "Daicho-kei" are arranged symmetrically with respect to the center of the circle. The optimum value of each "Keiraku" is linked with a solid line 200.

The inspector can understand the balance of the upper and lower half of a body, that of the left and right sides of a body, and that of "In" and "You" by comparing the optimum value with the reference (0 volt) shown by an inner circle 202 and/or comparing the optimum values of "Keiraku's" each other.

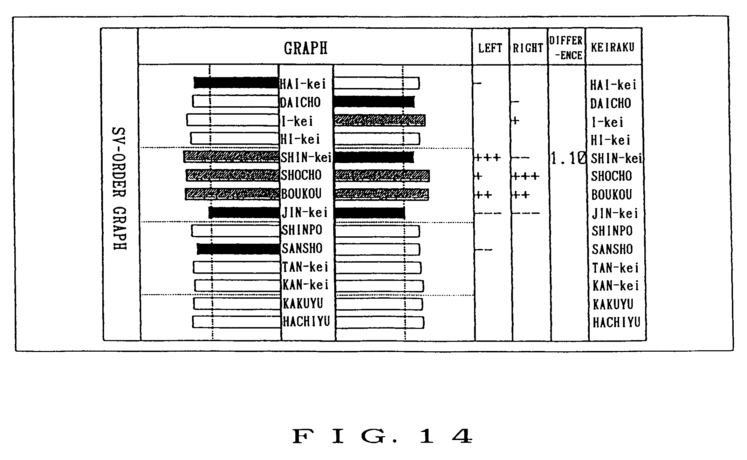

An example of bar graph is shown in Fig. 14. In this bar graph, the optimum values of respective "Keiraku's" are graphed along a circulated loop of "Keiraku's." According to the bar graph, it can be judged into which "Keiraku" in the circulated loop abnormality is concentrated. Also, "Keiraku's" are arranged in pairs each thereof being related to respective function of breathing, digestion, circulation, urination, control and excretion, so that it can be visually understood which "Keiraku" has abnormality. Abnormal "Keiraku" is shown by the following marks (+, -) in the screen with respect to the left and right sides of a body.

| "keiraku" in "Jitsu" (measurements are large) | |
| --- | --- |
| "Keiraku" having the first largest measurements | +++ |
| "Keiraku" having the second larger measurements | ++ |
| "Keiraku" having the third large measurements | + |

| "keiraku" in "Kyo" (measurements are small) | |
|---|---|
| "Keiraku" having the first smallest measurements | --- |
| "Keiraku" having the second smaller measurements | -- |
| "Keiraku" having the third small measurements | - |

Using these marks, it becomes easy for the inspector to judge abnormal "Keiraku's" even if the difference between measurements is very small. Furthermore, it becomes possible for the inspector to diagnose physical constitution and condition and to judge "Keiraku" to be cured. According to the present invention, the "Keiraku's" to be cured are judged by means of the diagnosing unit 132. For example, "Keiraku's" having large or small measurements are judged to be necessary for medical treatment.

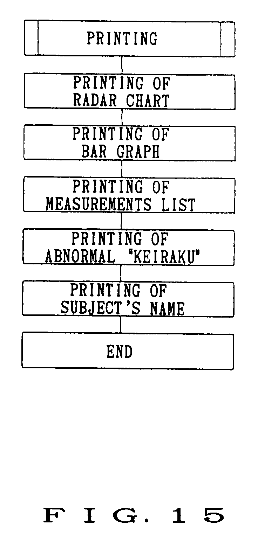

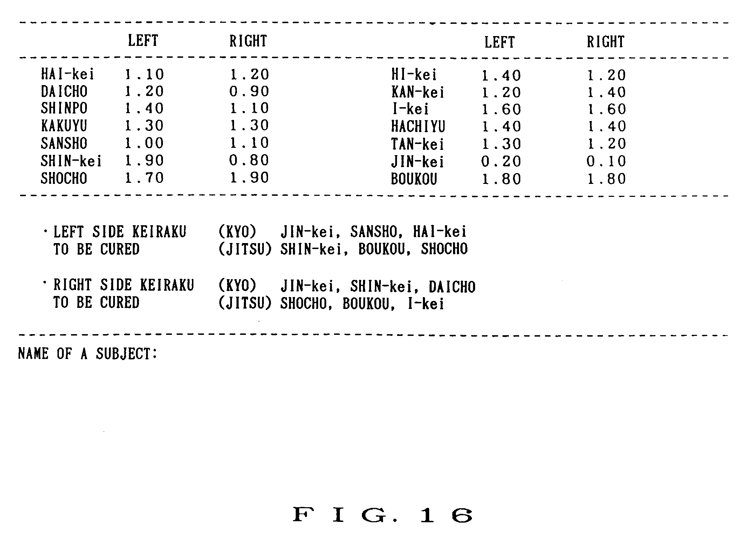

The data generated by the graph forming unit 130 and the diagnosing unit 132 are indicated on the display 116 and/or printed by the printer 118. Fig. 15 shows the flow chart of printing process. According to this printing process, it is possible to print some radar charts and bar graphs as indicated on the display, and a list of measurements and abnormal "Keiraku's." An example of the list of measurements and abnormal "Keiraku's" is shown in Fig. 16. In the list, 28 measurements are arranged in measuring order, and it can be understood that three "Keiraku's" having large measurements and three small measurements are printed as abnormal "Keiraku's" in both right and left sides of a body. Finally, the name of a subject is printed, thereby the process being ended.

The diagnosing device has been discussed in which "Seiketsu" points are selected as measuring points. However, measuring points are not limited to "Seiketsu" points, but other "Keiketsu" points or any point on the surface of a body may be selected.

The present invention is not limited to the embodiment, but it is clear for those who skilled in the art to realize various modifications within the scope of the invention. For example, the A/D converter can be provided not in the processing device but in the biological surface measuring device in order that the measuring device itself can output surface potential in digital.

Industrial Applicability

According to the biological surface potential measuring device of the present invention, precise measurements can be obtained because any point on the surface of a living body are measured without contacting electrodes to the surface of a living body.

Furthermore, measuring can be effected easily and the potential of any location on the surface of a living body can be measured, because a pickup is only contacted to a measuring point on the surface of a living body.

According to the diagnosing device of the present invention using the biological surface potential measuring device, it is possible to diagnose physical constitution and condition by determining abnormality of "Keiraku" in Oriental medicine.

The diagnosing device of the present invention comprises two functions such that optimum values are automatically calculated and "Keiraku" to be cured are automatically judged, then a series of operation from measuring to diagnosing can be automatically effected.

**Claims**

1. A device for measuring the surface potential of a living body, comprising :
    a pickup composed of a vibrating reed potential sensor for producing an AC voltage signal, the pickup being conducted to a surface of the living body ; and
    a detecting circuit for detecting the surface potential of the living body from the AC voltage signal supplied by the pickup.

2. The device of claim 1, wherein the pickup includes,
    a detecting electrode,
    a vibrator for vibrating the detecting electrode,
    a resistor connected to the detecting electrode, across thereof the AC voltage signal is generated.

3. The device of claim 2, wherein the detecting circuit includes,
    a bandpass filter for removing frequency components other than vibrating frequency generated by the vibrator from the AC voltage signal,

an AC amplifier for amplifying the AC voltage signal from the bandpass filter,

an oscillator for generating an oscillating voltage to vibrate the vibrator of the pickup,

a reference clock generator for generating a reference clock on the basis of the oscillating voltage supplied by the oscillator,

a rectifier, to which the amplified AC voltage signal and the reference clock are supplied, for synchronous-detecting the amplified AC voltage signal on the basis of the reference clock to generate a synchronous-detected signal and integrating the synchronous-detected signal to detect the surface potential on the surface of the living body, and

a DC amplifier for amplifying the detected potential to output it as an analog measurement.

4. A diagnosing device comprising :

a device for measuring the surface potential of a living body and generating the measured surface potential as analog measurements ;

a processing device for obtaining predetermined information from the surface potentials measured on a plurality of measuring points by the device for measuring the surface potential of the living body ;

a display for indicating the information on a screen ; and

a printer for printing the information.

5. The diagnosing device of claim 4, wherein the processing device includes,

an A/D converter for converting the analog measurements into digital measurements,

a sampling unit for sampling the predetermined number of the digital measurements every predetermined time interval,

an optimum value calculating unit for calculating optimum values on the basis of the sampled digital measurements,

a memory for storing the optimum values,

a graph forming unit for reading out the optimum values stored in the memory to form graphs, and

a diagnosing unit for diagnosing the living body on the basis of the optimum values read out from the memory.

6. The diagnosing device of claim 5, wherein measuring point on the surface of the living body is "Seiketsu" in Oriental medicine.

7. The diagnosing device of claim 6, wherein the graph forming unit forms radar charts and bar graphs, and the diagnosing device ldiagnoses the living body on the basis of whether optimum values are abnormal or not.

8

F I G. 1

F I G. 2

F I G . 3

116 DISPLAY

118 PRINTER

114 PROCESSING DEVICE

120 KEY BOARD

112 MEASUR -ING DEVICE

110

2

F I G. 4

FIG. 5

122 A/D CONVERTER
124 SAMPLING UNIT
126 OPTIMUM VALUE CALCULATING UNIT
128 MEMORY
130 GRAPH FORMING UNIT
132 DIAGNOSING UNIT
134 CONTROLLER
114
116 DISPLAY
118 PRINTER
MEASURING DEVICE
KEY BOARD

F I G . 6

SHOCHO-kei
SHIN-kei
SANSHO-kei
KAKUYU-kei
SHINPO-kei
DAICHO-kei
HAI-kei

BOUKOU-kei
JIN-kei
TAN-kei
HACHIYU-kei
I-kei
KAN-kei
HI-kei

EP 0 714 629 A1

```
┌─┬─────────────────────┬─┐
│ │     PROCESSING      │ │
└─┴─────────────────────┴─┘
┌───────────────────────┐
│      PREPROCESS       │
└───────────────────────┘
┌───────────────────────┐
│      NAME INPUT       │
└───────────────────────┘
┌───────────────────────┐
│      MEASUREMENT      │
└───────────────────────┘
┌───────────────────────┐
│      INDICATION       │
└───────────────────────┘
┌───────────────────────┐
│       PRINTING        │
└───────────────────────┘
┌───────────────────────┐
│         END           │
└───────────────────────┘
```

## F I G . 7

```
┌─┬─────────────────────┬─┐
│ │     PREPROCESS      │ │
└─┴─────────────────────┴─┘
┌───────────────────────┐
│    DESIGNATION OF     │
│    INCLUDED FILE      │
└───────────────────────┘
┌───────────────────────┐
│    DECLARATION OF     │
│ VARIABLE AND SUBMODULE│
└───────────────────────┘
┌───────────────────────┐
│        SCREEN         │
│    INITIALIZATION     │
└───────────────────────┘
┌───────────────────────┐
│  COLOR DESIGNATION    │
│     OF PALETTE        │
└───────────────────────┘
┌───────────────────────┐
│    INDICATION OF      │
│    SCREEN FRAME       │
└───────────────────────┘
┌───────────────────────┐
│         END           │
└───────────────────────┘
```

## F I G . 8

```
+-------------------------------+
| |      NAME INPUT         | |
+-------------------------------+
|      INPUT NAME OF            |
|      A SUBJECT               |
+-------------------------------+
|           END                 |
+-------------------------------+
```

F I G. 9

```
┌─────────────────────────────┐
│         MEASURING           │
└─────────────────────────────┘
┌─────────────────────────────┐
│      INDICATION OF          │
│    MEASURING SCREEN         │
└─────────────────────────────┘

┌─────────────────────────────┐
│      REPEAT FOR 28          │
│    MEASURING POINTS         │
└─────────────────────────────┘
┌─────────────────────────────┐
│      INDICATION OF          │
│    MEASURING POINT          │
└─────────────────────────────┘
┌─────────────────────────────┐
│   INPUT OF MEASURING        │
│   STARTING KEY              │
└─────────────────────────────┘
┌─────────────────────────────┐
│ 10 TIMES AUTOMATICALLY      │
│ MEASURING                   │
└─────────────────────────────┘
┌─────────────────────────────┐
│      INDICATION OF          │
│    END OF MEASURING         │
└─────────────────────────────┘
┌─────────────────────────────┐
│    CALCULATION OF           │
│    OPTIMUM VALUES           │
└─────────────────────────────┘

┌─────────────────────────────┐
│            END              │
└─────────────────────────────┘
```

F I G. 1 O

```
┌─────────────────────────────┐
│         INDICATION          │
└─────────────────────────────┘
┌─────────────────────────────┐
│       INDICATION OF         │
│        RADAR CHART          │
└─────────────────────────────┘
┌─────────────────────────────┐
│       INDICATION OF         │
│        BAR GRAPH            │
└─────────────────────────────┘
┌─────────────────────────────┐
│            END              │
└─────────────────────────────┘
```

# F I G. 1 1

HAND'S KEIRAKU     FOOT'S KEIRAKU

JOUSHO (BREATHING / DIGESTION)    HAI-kei → DAICHO-kei → I-kei → HI-kei ─┐

CHUSHO (CIRCULATION / URINATION)    └SHIN-kei→ SHOCHO-kei → BOUKOU-kei→ JIN-kei ─┐

KASHO (CONTROL / EXCRETION)    └SHINPO-kei→ SANSHO-kei → TAN-kei → KAN-kei → (RETURN TO HAI-kei)

       IN       YOU       IN       YOU

# F I G. 1 2

EP 0 714 629 A1

**SURFACE POTENTIAL MEASURED RESULT**

LEFT HAND — HAI-kei, SHIN-kei, SHINPO, SANSHO, SHOCHO, DAICHO

RIGHT HAND — HAI-kei, SHIN-kei, SHINPO, SANSHO, SHOCHO, DAICHO

LEFT FOOT — HI-kei, JIN-kei, KAN-kei, TAN-kei, BOUKOU, I-kei

RIGHT FOOT — HI-kei, JIN-kei, KAN-kei, TAN-kei, BOUKOU, I-kei

F I G. 13

EP 0 714 629 A1

| SV-ORDER GRAPH | GRAPH | | LEFT | RIGHT | DIFFER-ENCE | KEIRAKU |
|---|---|---|---|---|---|---|
| | HAI-kei | | — | | | HAI-kei |
| | DAICHO | | | — | | DAICHO |
| | I-kei | | | + | | I-kei |
| | HI-kei | | | | | HI-kei |
| | SHIN-kei | | +++ | — — | 1.10 | SHIN-kei |
| | SHOCHO | | + | +++ | | SHOCHO |
| | BOUKOU | | ++ | ++ | | BOUKOU |
| | JIN-kei | | — — — | — — — | | JIN-kei |
| | SHINPO | | | | | SHINPO |
| | SANSHO | | — — | | | SANSHO |
| | TAN-kei | | | | | TAN-kei |
| | KAN-kei | | | | | KAN-kei |
| | KAKUYU | | | | | KAKUYU |
| | HACHIYU | | | | | HACHIYU |

F I G. 1 4

```
┌──────────────────────────────┐
│┌────────────────────────────┐│
││         PRINTING           ││
│└────────────────────────────┘│
└──────────────────────────────┘
┌──────────────────────────────┐
│        PRINTING OF           │
│        RADAR CHART           │
└──────────────────────────────┘
┌──────────────────────────────┐
│        PRINTING OF           │
│         BAR GRAPH            │
└──────────────────────────────┘
┌──────────────────────────────┐
│        PRINTING OF           │
│     MEASUREMENTS LIST        │
└──────────────────────────────┘
┌──────────────────────────────┐
│        PRINTING OF           │
│    ABNORMAL "KEIRAKU"        │
└──────────────────────────────┘
┌──────────────────────────────┐
│        PRINTING OF           │
│      SUBJECT'S NAME          │
└──────────────────────────────┘
┌──────────────────────────────┐
│            END               │
└──────────────────────────────┘
```

F I G . 1 5

| | LEFT | RIGHT | | | LEFT | RIGHT |
|---|---|---|---|---|---|---|
| HAI-kei | 1.10 | 1.20 | | HI-kei | 1.40 | 1.20 |
| DAICHO | 1.20 | 0.90 | | KAN-kei | 1.20 | 1.40 |
| SHINPO | 1.40 | 1.10 | | I-kei | 1.60 | 1.60 |
| KAKUYU | 1.30 | 1.30 | | HACHIYU | 1.40 | 1.40 |
| SANSHO | 1.00 | 1.10 | | TAN-kei | 1.30 | 1.20 |
| SHIN-kei | 1.90 | 0.80 | | JIN-kei | 0.20 | 0.10 |
| SHOCHO | 1.70 | 1.90 | | BOUKOU | 1.80 | 1.80 |

· LEFT SIDE KEIRAKU     (KYO)     JIN-kei, SANSHO, HAI-kei
  TO BE CURED           (JITSU)  SHIN-kei, BOUKOU, SHOCHO

· RIGHT SIDE KEIRAKU    (KYO)     JIN-kei, SHIN-kei, DAICHO
  TO BE CURED           (JITSU)  SHOCHO, BOUKOU, I-kei

NAME OF A SUBJECT:

F I G. 1 6

EP 0 714 629 A1

<div style="text-align:center">

**INTERNATIONAL SEARCH REPORT**

</div>

| International application No. |
| --- |
| PCT/JP95/00993 |

**A. CLASSIFICATION OF SUBJECT MATTER**

Int. Cl$^6$  A61B5/04

According to International Patent Classification (IPC) or to both national classification and IPC

**B. FIELDS SEARCHED**

Minimum documentation searched (classification system followed by classification symbols)

Int. Cl$^6$  A61B5/04, G01R29/12

Documentation searched other than minimum documentation to the extent that such documents are included in the fields searched

| | |
| --- | --- |
| Jitsuyo Shinan Koho | 1945 – 1994 |
| Kokai Jitsuyo Shinan Koho | 1971 – 1994 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP, 63-165772, A (Brews T. Williams), July 9, 1988 (09. 07. 88) & US, 4878017, A & US, 4928057, A | 1 – 7 |
| Y | JP, 3-268738, A (Nippondenso Co., Ltd.), November 29, 1991 (29. 11. 91)(Family: none) | 1 – 7 |
| Y | JP, 2-63433, A (Matsushita Electric Ind. Co., Ltd.), March 2, 1990 (02. 03. 90)(Family: none) | 1 – 7 |
| Y | JP, 62-148645, A (Hiroshi Motoyama), July 2, 1987 (02. 07. 87)(Family: none) | 4 – 7 |

☐ Further documents are listed in the continuation of Box C.    ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| August 1, 1995 (01. 08. 95) | August 29, 1995 (29. 08. 95) |

| Name and mailing address of the ISA/ | Authorized officer |
| --- | --- |
| Japanese Patent Office | |
| Facsimile No. | Telephone No. |

Form PCT/ISA/210 (second sheet) (July 1992)